(19) **Europäisches Patentamt**

**European Patent Office**

**Office européen des brevets**

(11) **EP 1 498 426 A1**

(12) **EUROPEAN PATENT APPLICATION**

(43) Date of publication:
**19.01.2005 Bulletin 2005/03**

(51) Int Cl.[7]: **C07K 16/00**, C12N 5/00
// C07K16/10

(21) Application number: **04016838.7**

(22) Date of filing: **16.07.2004**

| | |
|---|---|
| (84) Designated Contracting States: **AT BE BG CH CY CZ DE DK EE ES FI FR GB GR HU IE IT LI LU MC NL PL PT RO SE SI SK TR** Designated Extension States: **AL HR LT LV MK** | (71) Applicant: **CCL Holdings Co., Ltd. Taipei (TW)** |
| (30) Priority: **16.07.2003 US 622003** | (72) Inventor: **Chin, Li-Te Hsin-Chu 300 (TW)** (74) Representative: **VOSSIUS & PARTNER Siebertstrasse 4 81675 München (DE)** |

(54) **Preparation of fully human antibodies**

(57) The present invention provides a method of preparing fully human antibodies that recognize a pre-determined antigen without relying on human donors that have already been exposed to the antigen. To this end, lymphocytes from naive human donors are immunized *in vitro* with the antigen of interest, and cells that produce antibodies against the antigen are identified.

Since the lymphocytes are immunized *in vitro* rather than *in vivo*, it is possible to control which antigen, or which part of the antigen, would be recognized by the antibody. A preferred antigen is gp120 of HIV, particularly the co-receptor binding region of gp120.

EP 1 498 426 A1

**Description**

**FIELD OF THE INVENTION**

This invention relates to methods of preparing fully human antibodies against any antigen of interest, as well as the resulting antibodies.

**REFERENCES**

U.S. Patent No. 5,023,252.
U.S. Patent No. 6,190,871.
U.S. Patent No. 6,228,361.
U.S. Patent No. 6,261,558.
U.S. Patent No. 6,391,635.
U.S. Patent No. 6,395,275.
U.S. Patent No. 6,514,496.
U.S. Patent No. 6,592,904.
Breedveld FC. (2000) Therapeutic monoclonal antibodies. Lancet 355: 735-40.
Chin LT, Hinkula J, Levi M, Ohlin M, Wahren B, Borrebaeck CAK. (1994) Site-directed primary *in vitro* immunization: Production of HIV-1 neutralizing human monoclonal antibodies from sero-negative donors. Immunology 81: 428-434.
Chin LT, Malmborg AC, Kristensson K, Hinkula J, Borrebaeck, CAK. (1995) Mimicking the humoral immune response *in vitro* results in antigen-specific isotype switching by autologous T helper cells. Eur. J. Immunol. 25:657-663.
Chin LT, Cheng, JY, Lu, SC, Chang, ACH, Chu, CH, Meng, CL. ' (2001) Establishment and evaluation of mouse-human heteromyeloma cell lines obtained by electrofusion for immortalizing human immunoglobulins. J Biomed Lab Sci 13:117-123.
Demotz S, Lanzavecchia A. Eisel U. Niemann H. Widmann C. Corradin, G. (1989) Delineation of several DR-restricted tetanus toxin T cell epitopes. J Immunol. 142:394-402.
Dragic, T. (2001) An overview of the determinants of CCR5 and CXCR4 co-receptor function. J. General Virology 82:1807-1814.
Dueñas M, Chin LT, Malmborg AC, Casalvilla R. Ohlin M, Borrebaeck CAK. (1996) *In vitro* immunization of naive human B cells yields high affinity immunoglobulin G antibodies as illustrated by phage display. Immunology 89 (1):1-7.
Hahn B. H., Shaw G. M., Taylor M. E., Redfield R. R., Markham P. D., Salahuddin S. Z., Wong-Staal F., Gallo R. C., Parks E. S., Parks W. P. (1986) Genetic variation in HTLV-III/LAV over time in patients with AIDS or at risk for AIDS. Science 232:1548-1554.
Hahn BH, Gonda MA, Shaw GM, Popovic M, Hoxie JA, Gallo RC, Wong-Staal F. (1985) Genomic diversity of the acquired immunodeficiency syndrome virus HTLV-III: different viruses exhibit greatest divergence in their envelope genes. Proc Natl Acad Sci (USA) 82:4813-4817.
Hill CM, Deng H, Unutmaz D, Kewalramani VN, Bastiani L, Gomy MK, Zolla-Pazner S, Littman DR. (1997) Envelope glycoproteins from human immunodeficiency virus types 1 and 2 and simian immunodeficiency virus can use human CCR5 as a coreceptor for viral entry and make direct CD4-dependent interactions with this chemokine receptor. J Virol. 71:6296-304.
Korber BTM, Brander C, Haynes BF, Koup R, Kuiken C, Moore JP, Walker BD, Watkins DI (Editors). *HIV Molecular Immunology* 2001. Publisher: Los Alamos National Laboratory, Theoretical Biology and Biophysics, Los Alamos, New Mexico. LA-UR 02-4663.
Kuhmann S. E., Platt E. J., Kozak S. L., Kabat D. (2000) Cooperation of multiple CCR5 coreceptors is required for infections by human immunodeficiency virus type 1. J Virol 74:7005-15.
Lee B, Sharron M, Montaner LJ, Weissman D, Doms RW. (1999) Quantification of CD4, CCR5, and CXCR4 levels on lymphocyte subsets, dendritic cells, and differentially conditioned monocyte-derived macrophages Proc Natl Acad Sci USA. 96: 5215-5220.
Modrow S., Hahn B. H., Shaw G. M., Gallo R. C., Wong-Staal F., WolfH. (1987) Computer assisted analysis of envelope protein sequences of seven human immunodeficiency virus isolates: predictions of antigenic epitopes in conserved and variable regions. J. Virol 61:570-578.
Nermut MV, Grief C, Hashmi S, Hockley DJ. (1993) Further evidence of icosahedral symmetry in human and simian immunodeficiency virus. AIDS Res. Hum. Retroviruses 9:929-38.
Ohlin M, Kristensson K, Carlsson R, Borrebaeck CA. (1992) Epstein-Barr virus-induced transformation of human B lymphocytes: the effect of L-leucyl-L-leucine methyl ester on inhibitory T cell populations. Immunol Lett. 34:

221-8.

Ohlin M., Danielsson L, Carlsson R, Borrebaeck, CAK. (1989) The effect of leucyl-leucine methyl ester on proliferation and Ig secretion of EBV-transformed human B lymphocytes. Immunology 66: 485-490.

Shiino T, Kato K, Kodaka N, Miyakuni T, Takebe Y, Sato H. (2000) A group of V3 sequences from human immunodeficiency virus type 1 subtype E non-syncytium-inducing, CCR5-using variants are resistant to positive selection pressure. J Virol. 74(3):1069-78.

Staudinger R, Phogat SK, Xiao X, Wang X, Dimitrov DS, Zolla-Pazner S. (2003) Evidence for CD4-enchanced signaling through the chemokine receptor CCR5. J Biol Chem. 278:10389-92.

Thomson MM, Perez-Alvarez L, Najera R. (2002) Molecular epidemiology of HIV-1 genetic forms and its significance for vaccine development and therapy. Lancet Infect Dis 2:461-71.

van Dijk MA, van de Winkel JG. (2001) Human antibodies as next generation therapeutics. Curr Opin Chem Biol. 5(4):368-74.

Weiner LM. (1999) An overview of monoclonal antibody therapy of cancer. Semin Oncol 26:41-50.

Wu H, Myszka DG, Tendian SW, Brouillette CG, Sweet RW, Chaiken IM, Hendrickson WA. (1996) Kinetic and structural analysis of mutant CD4 receptors that are defective in HIV gp120 binding. Proc Natl Acad Sci USA. 93: 15030-5.

Wyatt R, Moore J, Accola M, Desjardin E, Robinson J, Sodroski J. (1995) Involvement of the V1/V2 variable loop structure in the exposure of human immunodeficiency virus type 1 gp120 epitopes induced by receptor binding. J Virol. 69:5723-33.

Zafiropoulos A, Andersson E, Krambovitis E, Borrebaeck CAK. (1997) Induction of antigen-specific isotype switching by *in vitro* immunization of human naive B lymphocytes. J Immunol Methods. 200(1-2):181-90.

All of the publications, patents and patent applications cited above or elsewhere in this application are herein incorporated by reference in their entirety to the same extent as if the disclosure of each individual publication, patent application or patent was specifically and individually indicated to be incorporated by reference in its entirety.

## BACKGROUND OF THE INVENTION

Humanized monoclonal antibodies have been successfully used for therapeutic purposes against various diseases (Breedveld, 2000). These diseases are traditionally infectious diseases, such as infections by respiratory syncytial virus (RSV). Recently, however, antibodies are increasingly used in the therapy of many other disorders, including autoimmune disorders and malignancies like metastatic breast cancer, non-Hodgkin's lymphoma, chronic lymphocytic leukemia and acute myeloid leukemia (Breedveld, 2000; Weiner, 1999). Prophylactic use against organ rejection or blood clotting during angioplasty has also been achieved.

Humanized antibodies are typically prepared by replacing regions of mouse antibodies that are unimportant for antigen specificity with a human counterpart. The resulting antibodies thus have residual murine sequences which, when administered to a human patient, often elicit immunological responses in the patient (human anti-mouse response). Therefore, it is desirable to prepare fully human antibodies that are void of non-human sequences. Fully human antibodies have been reported, such as by constructing and screening a human antibody library using the phage display technique, by grafting lymphocytes from immunized human donors into severe combined immunodeficient (SCID) mice, or by engineering transgenic mice harboring human immunoglobulin genes (van Dijk et al., 2001). Fully human antibodies against pathogens have also been isolated by extensive screening of cord blood, which contains a natural polyreactive IgM repertoire (see, *e.g.*, U.S. Patent No. 6,391,635). These methods, however, either produce antibodies with low affinities or depend on human donors with a desired immune response.

Since chemotherapy against HIV is often hampered by severe side effects and drug resistance, fully human antibodies against HIV would be therapeutically valuable. Of particular interest are antibodies that recognize gp120, the envelope glycoprotein of HIV. HIV entry into target cells is initiated by the sequential interaction of gp120 with CD4 and a co-receptor (CCR5 or CXCR4) on the target cell (Dragic, 2001). Gp120 is composed of five constant regions (C1-C5) and five variable loops (V1-V5). Binding of CD4 to gp120 generates a large bonding energy that causes a conformational change of gp120, exposing a co-receptor binding site (V3-C4 of gp120). Subsequently, the co-receptor binds to gp120, leading to the fusion of viral and cellular membranes, followed by virus entry into the cell. Therefore, antibodies recognizing the CD4 binding site or the co-receptor binding site interfere with HIV entry and prevent the virus from replication in the cell.

Fully human antibodies against gp120 have been reported. U.S. Patent No. 6,190,871 discloses four neutralizing huMAbs to gp120 derived from chronically infected patients, but all of them react with the CD4 binding region. U.S. Patent No. 6,228,361 reveals another IgG1 neutralizing huMAb, derived from a seropositive patient, also reactive to the CD4 binding region of gp120. In addition to whole antibody molecules, immunoglobulin fragments (Fab) acting on the same region were obtained from the bone marrow of an infected donor (U.S. Patent Nos. 6,261,558 and 6,395,275).

However, therapeutic antibodies recognizing the co-receptor binding site, rather than the CD4 binding site, are preferred for the following reasons. Firstly, ultrastructural studies suggest that a mature HIV particle possesses about 72 spikes containing gp120 knobs (Nermut et al., 1993). The average number of CD4 molecules on a peripheral lymphocyte was reported to be $65,330 \pm 6,049$ (Lee et al., 1999). Moreover, the affinity between CD4 and gp120 is high, with a Kd in the low nanomolar range, *i.e.,* 0.2 to 30 nM (Wu et al., 1996; Hill et al., 1997). Taken together, the overall strength of binding (avidity) between CD4 and the CD4 binding site of gp120, both abundant, is extremely high. Thus, not only would it take a vast amount of antibodies to block the interaction between the CD4 binding site of gp120 and CD4, but also in particular, antibodies with an extreme high affinity would be required to overcome the avidity described above. In contrast, the co-receptor binding site is not effectively exposed until gp120 is already bound to CD4. Therefore, it will take substantially fewer antibodies that bind to the co-receptor binding site to block binding of HIV to target cells, and the affinity of these antibodies do not have to be particularly high to efficiently inhibit HIV infection.

A further problem in preparing therapeutic antibodies targeting gp20 is that the primary structure, *i.e.*, amino acid sequence, of gp120 is highly variable among different HIV-1 strains (Kuhmann et al., 2000; Hahn et al., 1985; Modrow et al., 1987; Thomson et al., 2002). In fact, even sequential isolates from a single patient show variation (Hahn et al., 1986). Therefore, fully human antibodies that are capable of recognizing conformational epitopes on gp120, which can be used for therapeutic and/or preventive purposes against multiple HIV strains, are desirable. The ideal antibodies would recognize the co-receptor binding region of gp120. particularly conformational epitopes.

## SUMMARY OF THE INVENTION

The present invention provides a method of preparing fully human antibodies that recognize a pre-determined antigen without relying on human donors that have already been exposed to the antigen. To this end, lymphocytes from naive human donors are contacted *in vitro* with the antigen of interest, and cells that produce antibodies against the antigen are identified. Since the lymphocytes are immunized *in vitro* rather than *in vivo,* it is possible to control which antigen, or which part of the antigen, would be recognized by the antibody. Thus, this method is particularly useful in the preparation of antibodies against life-threatening microorganisms, since it is not necessary to immunize an animal, let alone a human, with a life-threatening microorganism.

A preferred antigen is the gp120 glycoprotein of HIV, or an immunogenic fragment thereof. More preferably, the antigen is a peptide comprising the co-receptor binding site of gp120. Due to antigenic variation , it has long been a problem to produce broad-spectrum antibodies against gp120, and existing antibodies against gp120 typically recognize the gp120 of only one subtype or even one clade of HIV. The present invention further provides a method of generating antibodies that recognize at least two different antigens. Thus, by immunizing the lymphocytes with one antigen and screening the immunized lymphocytes with another antigen, fully human antibodies recognizing both antigens can be obtained.

To prolong the ability of antibody production, the antibody-producing cells can optionally be subject to various further treatment. For example, the cells can be fused with heteromyeloma cells to form trioma cells, which can live a long time and stably produce antibodies.

Accordingly, one aspect of the present invention provides a method of preparing a fully human antibody recognizing an antigen, comprising:

(a) providing a group of lymphocytes obtained from a naive human donor;
(b) immunizing said lymphocytes with the antigen *in vitro;*
(c) fusing the immunized lymphocytes with a heteromyeloma cell line to form trioma cells;
(d) identifying trioma cells that produce the antibody that recognizes the antigen; and
(e) collecting the antibody produced from the trioma cells identified in step (d).

The method may further comprise the step of removing CD8[+] cells and CD56[+] cells from said lymphocytes prior to step (b). Additionally or alternatively, the method may further comprise screening the trioma cells of step (c) with a second antigen prior to step (d), thereby identifying antibodies that recognize both the antigen and the second antigen.

When the trioma cells are cultured in clonal conditions, monoclonal antibodies can be prepared using the present invention. Alternatively, polyclonal antibodies can be prepared from a group of antibody-producing cells obtained using the present invention, which are not cultured as individual clones. The antibody preferably recognizes the antigen with a Kd of about 100 nM or less, about 30 nM or less, about 10 nM or less, about 3 nM or less, or about 1 nM or less. The antibody is preferably an IgG antibody, particularly IgG1.

Also provided are methods for preparing antibody producing cells that produce fully human antibodies, such as the trioma cells described above. In particular, the cells are capable of producing the antibodies for a prolonged time. such as at least about 3, 6, 9, 12, 15, 18, 21, or 24 months in cell culture.

The antigen employed in the present invention is preferably an HIV antigen, more preferably the HIV gp120 molecule, or a fragment of the gp120 molecule. In particular, the antigen is a peptide derived from the co-receptor binding region of gp120.

Another aspect of the present invention provides fully human antibodies prepared according to the methods of the invention. In particular, the antibodies can recognize an HIV antigen, such as gp120, and preferably recognize the antigen from two different HIV strains. The antibody may, more preferably, recognize three or more HIV strains, subtypes, or clades. The fully human antibody preferably recognizes the co-receptor binding regions from at least two strains of HIV. For example, the antibody recognizes at least two antigens that comprise different co-receptor binding regions of gp120, such as those selected from the group consisting of SEQ ID NOs:2-17. The antibodies are preferably IgG, particularly IgG1 antibodies. Pharmaceutical compositions comprising the antibodies are also provided, which may comprise a pharmaceutically acceptable carrier and/or excipient.

Yet another aspect of the present invention provides a method for preventing, treating or ameliorating HIV infections comprising administering an effective amount of the antibody of the present invention, or a composition comprising the same, to a subject in need thereof. Specifically, the method can be used to prevent, treat or ameliorate AIDS.

A further aspect of the present invention provides a method of preparing a fully human antibody recognizing at least two different antigens, comprising:

(a) providing a group of lymphocytes from a naive human donor;
(b) immunizing said lymphocytes with a first antigen *in vitro*;
(c) fusing the immunized lymphocytes with a heteromyeloma cell line to form trioma cells;
(d) screening the trioma cells with a second antigen to identify trioma cells that produce antibodies which recognize both the first and the second antigens; and
(e) collecting the antibody produced by the trioma cells identified in step (d).

The first and the second antigens may be any antigens of interest. For example, the first and second antigens may be, independently, selected from the group consisting of peptides, proteins, glycoproteins, carbohydrates, lipids, glycolipids, cells, viruses, bacteria, fungi, prions, and parasites. The antigens are preferably derived from a microorganism, such as surface antigens derived from two different strains of a microorganism. The microorganism is preferably HIV, and the antigen is preferably gp120. Thus, the first and the second antigens may be gp120 molecules from different strains of HIV, particularly the co-receptor binding regions thereof.

Another aspect of the present invention provides a method of increasing the efficiency of *in vitro* immunization of lymphocytes with an antigen, comprising:

(a) providing a population of lymphocytes;
(b) removing $CD8^+$ and $CD56^+$ cells from said population; and
(c) contacting said population of lymphocytes with the antigen *in vitro.*

The $CD8^+$ and $CD56^+$ cells can be removed using any method established in the art. For example, these cells can be removed using antibodies that are specific for CD8 and CD56, respectively. In one embodiment, these antibodies are attached to magnetic beads.

A further aspect of the present invention provides a cell population prepared by a method comprising:

(a) providing lymphocytes from a naive human donor;
(b) removing $CD8^+$ and $CD56^+$ cells from said lymphocytes; and
(c) contacting the cells of step (b) with an antigen *in vitro*; resulting in cells that produce antibodies that recognize said antigen.

The lymphocytes can be provided as peripheral blood mononuclear cells. The cell population can be further diluted and cultured as individual clones, and clones that produce the desired antibodies can be identified. Thus, also provided is an antibody-producing cell prepared by culturing the cell population described above under clonal conditions and isolating clones that produce antibodies that recognize said antigen. The antibody-producing cells may be infected with EBV or fused with a fusion partner. Preferably, the cells are capable of producing the antibodies for a prolonged time, such as at least about 3, 6, 9, 12, 15, 18, 21, or 24 months in cell culture. The antigen is preferably an HIV antigen, more preferably an antigen derived from gp120, and most preferably the co-receptor binding region of gp120. In particular, the cells are capable of producing antibodies that recognize the gp120 molecules from at least two different HIV strains.

## BRIEF DESCRIPTION OF THE DRAWINGS

### Figure 1

Representative ELISA reactivity profile of LTC-gp120-IgG1k huMAb against tetanus toxoid, human transferrin, bovine serum albumin, and native and 10M urea-denatured recombinant gp120 derived from IIIB and MN strains of HIV-1.

### Figure 2

ELISA reactivity profile of LTC-gp120-IgG1k with synthetic peptides derived from various regions of gp120 and virus subtypes.

### Figure 3

HIV-1 neutralization assay using huMAb LTC-gp120-IgG1k. Neutralization was measured as relative inhibition of the reverse transcriptase (RT) activity of the virus. The HIV strains used were IIIB (■) and MN (○). The human myeloma protein (Δ) was used as a control in the place of the test antibody.

### Figure 4

Competitive ELISA analysis showing reactivity of LTC-gp120-IgG1k with V3B(IIIB) (■) and V3B(MN) (○) peptides. The peptide TT (Δ) was used as a control. The mean ELISA reading obtained from LTC-gp120-IgG1k binding to gp120(IIIB) and gp120(MN) was set as 100% in A and B, respectively. In the presence of increasing concentrations of competing peptides, antibody binding to gp120(IIIB) or gp120(MN) decreased.

## DETAILED DESCRIPTION OF THE INVENTION

The present invention provides a method of preparing fully human antibodies that recognize a pre-determined antigen without relying on human donors that have already been exposed to the antigen. To this end, lymphocytes from naive human donors are immunized *in vitro* with the antigen of interest, and cells that produce antibodies against the antigen are identified and selected. Since the lymphocytes are immunized *in vitro* rather than *in vivo,* it is possible to control which antigen, or which part of the antigen, would be recognized by the antibody. A preferred antigen is gp120 of HIV, particularly the co-receptor binding region of gp120.

Prior to describing the invention in further detail, the terms used in this application are defined as follows unless otherwise indicated.

### Definitions

A "fully human antibody" is an antibody containing exclusively human sequences. The antibody is preferably a monoclonal antibody.

A "naive human donor" is a human who has not been exposed to an antigen of interest and serves as the source of immune cells or factors. A naive donor does not contain detectable circulating antibodies against the antigen of interest. Typically naive human donors are healthy, regular blood donors who are consistently screened negative of anti-HIV antibodies.

"Immunize" a cell or an animal with an antigen refers to the action of exposing the cell or the animal to the antigen. The cell or animal can be immunized in any manner that leads to contact between the cell or the animal with the antigen.

A "heteromyeloma cell line" is a cell line derived from fusion of two different myeloma cells. The two different myeloma cells are preferably a human myeloma cell and a murine myeloma cell. Heteromyeloma cell lines are known in the art. For example, U.S. Patent No. 6,228,361 and Chin et al., 2001 describe the preparation, characterization and use of various heteromyeloma cell lines.

A "trioma cell" is the fusion product of a cell with a heteromyeloma cell.

A "fusion partner" is a cell that can be used to fuse with an antibody-producing cell for a beneficial purpose. Typically, the fusion leads to prolonged antibody production. Thus, without fusion to the fusion partner, the antibody-producing cell ceases to produce antibodies in culture. Upon fusion to the fusion partner, however, fused cells can be selected that produce antibodies in culture for at least about 3 months, preferably at least about 6, 9, 12, 18, 24 months or more. Fusion partners include, but are not limited to, myeloma cells and heteromyeloma cells.

"Treating or ameliorating" a disease or medical condition means reducing or eliminating the symptoms of the disease or medical condition, or slowing down the progress of the disease/medical condition. The reduction is preferably at least about 10%, more preferably at least about 20%, 30%, 40%, 50%, 60%, 70%, 80% or 90%.

"Preventing" a disease or medical condition means taking a measure in a subject who shows no symptoms of the disease or medical condition, wherein, as a result, the subject does not develop the disease/medical condition or develops the disease/medical condition to a lesser extent.

An "effective amount" is an amount of an agent that is sufficient to result in the intended effect. For example, for an antibody used to treat or ameliorate a disease, an effective amount is an amount of the antibody sufficient to reduce or eliminate the symptoms of the disease, or to slow down the progress of the disease.

A "sample" is an aliquot or a representative portion of a substance. material, or population. For example, a sample may be a sample of water, sewage, oil, sand, blood, biological tissue, urine or feces.

A "biological sample" is a sample collected from a biological subject, such as an animal, plant or microorganism.

**Method**

The present invention provides a method for preparing a fully human antibody without having to immunize any animal, including human. To generate the antibody, lymphocytes from a naive human donor are contacted *in vitro* with the antigen of interest. The immunized lymphocytes are then cultured under clonal conditions, and clones that produce the desired antibodies are identified. For the ease of cloning and characterization of the antibodies, the immunized cells may optionally be infected with EBV. Alternatively or in addition, the immunized cells may also be fused to a fusion partner, particularly a heteromyeloma cell line, for long-term and stable production of antibodies.

The co-receptor binding site of HIV gp120 was employed as the antigen to demonstrate the present invention. Thus, peptides were synthesized according to the sequences of the V3 loop of gp120 from different subtypes of HIV (Example 1). As discussed above, the gp120 sequences are highly variable. Therefore, a variety of peptides were prepared, including the consensus sequences from Subtypes A-H, J, K, and O1-O4, as well as the sequences from two different clades of the B Subtype, MN and IIIB (Table 1). To enhance immune responses against the antigen, the gp120 V3 Subtype B MN strain sequence was also linked to a T-cell epitope sequence to form a hybrid antigen (Table 2).

The peptide antigen was added *in vitro* to peripheral blood mononuclear cells (PBMC) from naive human donors in a two-step immunization procedure (Chin et al., 1995; Zafiropoulos et al., 1997) as described in Example 2. Traditionally, cells to be immunized *in vitro* are treated with L-leucyl-L-leucine methyl ester (LeuLeuOMe) to remove the cells that may inhibit the immunization process. The present inventor contemplated that removal of the $CD8^+$ and $CD56^+$ cells from PBMC prior to immunization would increase the efficiency of *in vitro* immunization. Thus, the effect of the traditional treatment (LeuLeuOMe) was compared to that of removing $CD8^+$ cells, $CD56^+$ cells, or both $CD8^+$ and $CD56^+$ cells. Indeed, when both $CD8^+$ and $CD56^+$ cells were removed, the number of specific antibody-producing cells was significantly higher (Table 3). Therefore, it is preferred that $CD8^+$ and $CD56^+$ cells are removed from the lymphocytes prior to immunization, optionally in combination with the LeuLeuOMe treatment.

After immunization, the cells are screened for production of antibodies with the desired specificity. This screening step can be performed by any method known in the art, typically by using an enzyme linked immunosorbent assay (ELISA) employing the antigen that the cells are immunized with. In Example 3, an alternative method is described, wherein the co-receptor binding region from one clade of HIV, the MN strain of Subtype B, was used to immunize the lymphocyte, and the corresponding region from another clade of HIV, the IIIB strain of Subtype B, was used to screen the immunized cells. Thus, the resulting antibody recognizes the gp120 co-receptor binding regions of both strains. In fact, with minor exceptions, the antibody recognized almost all the V3 consensus peptides listed in Table 1. Therefore, the present invention provides broad-spectrum antibodies that can be used in diagnostic and therapeutic applications for pathogens that undergo substantial antigenic variation. Further provided is method for generating fully human antibodies that recognize at least two antigens, a first antigen and a second antigen, comprising contacting a group of lymphocytes from a naive human donor with the first antigen and screening for antibody-producing cells with a second antigen. Antibodies produced by the cells thus identified can then be collected and reacted with the first antigen to confirm that the antibodies recognize both antigens.

Furthermore, although the antibody described above reacted strongly with either antigen from the MN or the IIIB strain, when the antigens were denatured, the reactivity was significantly lower. These results indicate that the antibody recognizes a conformational epitope shared by the antigens, rather than the linear sequence of either antigen. Thus, the present invention also provides a method of preparing fully human antibodies specific for conformational epitopes, particularly conformational epitopes that are shared by two or more antigens.

Instead effusion, the *in vitro* immunized cells can be used to construct an antibody library, and the antibodies of interest are then identified from this library. Thus, after *in vitro* immunization, antibody-producing cells can be identified with the antigen (the cells at this stage can be optionally infected with EBV). A phage-display library is then constructed using these antibody-producing cells, and the phages containing the antibody fragment of interest can be identified by screening this library with the antigen. The methods of constructing phage display libraries are known in the art (see, *e.g.*, Duenas et al., 1996).

This embodiment is particularly useful when one antigen is used to immunize the cells and a second antigen is used to screen for antibodies. For example, the lymphocytes are immunized with the first antigen, antibody-producing cells are identified by reacting the immunized cells with the second antigen, and a phage-display library can be constructed using the antibody-producing cells. The library is then screened with the antigens to identify the clones containing the antibody fragments of interest. To identify clones that recognize an even broader range of antigens, a third antigen may be used to screen the phage library. The specificity of any clone that recognizes the third antigen can then be determined with the first and the second antigens. Antibody fragments identified in this manner have a high likelihood of recognizing all three antigens by binding to a confonnational epitope shared among them.

Another aspect of the present invention provides a therapeutic method for treating a subject having a disease which comprises administering to the subject an antibody prepared according to the present invention, wherein the antibody is capable of treating or ameliorating the disease. Similarly, the antibody may be used to prevent a person from contracting the disease. The person may, for example, belong to a high-risk group for the disease as being genetically or otherwise predisposed, live in an area wherein a microbial infection is spreading, or have to contact pathogens frequently due to his or her occupation. Antibodies useful for practice of this aspect can be determined by methods known in the art. For example, the ability of an antibody to neutralize a pathogen or toxin can be measured as indicative of its capability in the prevention, treatment or amelioration. Preferably, an amount of the antibody is used to achieve a neutralization of at least about 50%, more preferably at least about 60%, 70%, 80% or 90%, and most preferably at least about 95%.

Preferably, the disease is tumor or an infectious disease caused by a microorganism. The tumor is preferably selected from the group consisting of hemopoietic maligencies, *e.g.*, lymphomas, leukemias, and myelomas; carcinomas such as adenocarcinomas, which may have a primary tumor site in the esophagus, lung, breast, ovary, liver, endometrium, cervix, colon, pancreas, prostate, stomach, intestines, rectum, or uterus; and squamous cell carcinomas, which may have a primary origin in the oral cavity, tongue, larynx, esophagus, lungs, skin, bladder, cervix, eyelid, conjunctiva, vagina, etc. Other classes of tumors that may be treated include sarcomas, *e.g.*, fibrosarcoma, myogenic sarcomas; neuromas; melanomas; trophoblastic and germ cell tumors; neuroendocrine and neuroectodermal tumors. In particular, the tumor is selected from the group consisting of metastatic breast cancer, non-Hodgkin's lymphoma, chronic lymphocytic leukemia and acute myeloid leukemia.

The microorganism is preferably HIV. However, other human chronic viral infections, e.g, hepatitis B virus (HBV), hepatitis C virus (HCV), human T lymphotropic viruses type 1 and 2 (HTLV-1 and HTLV-2), parvovirus. human herpes viruses including herpes simplex virus (HSV) types 1 and 2, Epstein Barr virus (EBV), cytomegalovirus (CMV), human papilloma virus (HPV), varicella-zoster virus (VZV) as well as human herpes virus 6 (HHV-6) may be prevented, treated or ameliorated using the present invention. Infection with other agents that replicate intracellularly, such as pathogenic protozoa, *e.g.* trypanosomes, malaria and toxoplasma gondii; bacteria, *e.g.* mycobacteria, salmonella, Chlamydia trachomatis and listeria; and fungi, *e.g.* candida; may also become chronic and thus are good candidates for treatment/prevention using the present invention.

Other medical conditions to which the present invention can be applied include, for example, poisoning by toxins. The toxins include microbial and animal toxins, such as enterotoxins, exotoxins, endotoxins, gliotoxin, ochartoxin, aflatoxin and venoms.

The present invention further provides a method of blocking binding of HIV to human cells and a method of preventing infection of human cells by HIV which comprises contacting the human cells with an anti-HIV antibody prepared according to the present invention, such as the antibody described in Example 4. The human cells are preferably located in a human, wherein an effective amount of the antibody is administered to the human to block HIV binding or prevent infection by HIV.

The antibodies can be administered by any suitable method known in the art, such as via intravascular, intrathecal, intravenous, intramuscular, parenteral, subcutaneous, intramedullar, intraperitoneal, topical, oral, rectal, vaginal, nasal, pulmonary and intratumoral routes.

Also provided is a method of detecting in a sample the presence of HIV, comprising contacting a suitable sample with the antibody of the present invention so as to form an antibody-antigen complex between the antibody and any HIV present in the sample, and detecting the presence of any complex so formed. thereby detecting in the sample the presence of HIV. In one embodiment, the human antibody is labeled with a detectable marker. Suitable samples which are useful in this method include, but are not limited to biological fluids from a human subject such as blood, serum, plasma, urine, nasal mucosal discharge, oral mucosal discharge. vaginal mucosal discharge, semen, anal mucosal discharge and serosal fluids.

**Compositions**

Another aspect of the present invention provides a composition comprising a fully human antibody prepared according to the present invention. Preferably, the antibody binds to its antigen with a high affinity. The Kd is preferably about 100 nM or less, more preferably about 30 nM or less, yet more preferably about 10 nM or less, still more preferably about 3 nM or less, and most preferably about 1 nM or less. In particular, the antibody is capable of recognizing at least two related antigens, such as microbial antigens that are only different due to antigenic variation, or proteins encoded by alleles of the same gene.

This invention also includes pharmaceutical compositions that contain, as the active ingredient, one or more of the antibodies in combination with a pharmaceutically acceptable carrier or excipients. In preparing the compositions of this invention, the active ingredient/antibody is usually mixed with an excipient, diluted by an excipient or enclosed within such a carrier which can be in the form of a capsule, sachet, paper or other container. When the pharmaceutically acceptable excipient serves as a diluent, it can be a solid, semi-solid, or liquid material, which acts as a vehicle, carrier or medium for the active ingredient. Thus, the compositions can be in the form of solutions (particularly sterile injectable solutions), tablets, pills, powders, lozenges, sachets, cachets, elixirs, suspensions, emulsions, syrups, aerosols (as a solid or in a liquid medium), ointments containing, for example, up to 10% by weight of the antibody, soft and hard gelatin capsules, suppositories, and sterile packaged powders.

Some examples of suitable excipients include lactose, dextrose. sucrose, sorbitol, mannitol, starches, gum acacia, calcium phosphate, alginates, tragacanth, gelatin, calcium silicate, microcrystalline cellulose, polyvinylpyrrolidone. cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

For preparing solid compositions such as tablets, the principal active ingredient/antibody is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions (such PBS), suitably flavored syrups, aqueous or oil suspensions, and flavored emulsions with edible oils such as com oil, cottonseed oil, sesame oil. coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders (see, *e.g.,* U.S. Patent Nos. 6,514,496 and 6,592,904). The liquid or solid compositions may contain suitable pharmaceutically acceptable excipients as described herein. Preferably the compositions are administered by the injection or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

Another preferred formulation employed in the methods of the present invention employs transdermal delivery devices ("patches"). Such transdermal patches may be used to provide continuous or discontinuous infusion of the antibody of the present invention in controlled amounts. The construction and use of transdermal patches for the delivery of pharmaceutical agents is well known in the art. See, for example, U.S. Patent 5,023,252, herein incorporated by reference. Such patches may be constructed for continuous, pulsatile, or on demand delivery of pharmaceutical agents.

Other suitable formulations for use in the present invention can be found in *Remington's Pharmaceutical Sciences.*

Further provided are cells, particularly antibody-producing cells, that are prepared according to the present invention. Thus, the present invention provides a cell population prepared by isolating PBMC from a naive human donor, removing CD8+ and CD56+ cells from the PBMC, and contacting the resulting cells with an antigen. The present invention further provides cells isolated from the cell population described above, which cells are capable of producing antibodies that recognize the antigen. The cells may be infected with EBV or fused with a fusion partner, such as a myeloma cell line or a heteromyeloma cell line. The cells can typically produce the antibody in culture for at least about 3 months, more preferably for at least about 6, 9, 12, 15, 18, 21 or 24 months, and most preferably more than 24 months.

Preferably, the cells produce antibodies that recognize an HIV antigen. particularly gp 120 and more particularly the co-receptor binding region of gp120. Furthermore, the cells can preferably produce antibodies that recognize antigens from at least two strains, subtypes or clades of HIV. The antibodies are preferably IgG antibodies, particularly IgG1.

The following examples are offered to illustrate this invention and are not to be construed in any way as limiting the scope of the present invention.

## EXAMPLES

In the examples below, the following abbreviations have the following meanings. Abbreviations not defined have their generally accepted meanings.

| | |
|---|---|
| °C = | degree Celsius |
| hr = | hour |
| min = | minute |
| sec = | second |
| µM = | micromolar |
| mM = | millimolar |
| M = | molar |
| ml = | milliliter |
| µl = | microliter |
| mg = | milligram |
| µg = | microgram |
| Mab = | monoclonal antibody |
| huMab = | human monoclonal antibody |
| DMEM = | Dulbecco's modified Eagle's medium |
| MEM = | modified Eagle's medium |
| PBS = | phosphate buffered saline |
| HANKS = | Hanks balanced salt solution |
| FBS = | fetal bovine serum |
| HIV = | human immunodeficiency virus |
| HTLV = | human T-cell leukemia virus |
| HCV = | hepatitis C virus |
| HBsAg = | hepatitis B surface antigen |
| ALT = | alanine transferase |
| PBMC = | peripheral blood mononuclear cell |
| LeuLeuOMe = | L-leucyl-L-leucine methyl ester |

## MATERIALS AND METHODS

*Culture materials*

The culture medium used in this disclosure, unless otherwise indicated, was RPMI-1640 (HyQ™; HyClone, Logan, UT), supplemented with 1 x non-essential amino acids (Life Technologies, Grand Island, NY), 10% fetal bovine serum (FBS; Life Technologies) and 50 mg/ml of gentamycin and kanamycin (China Chemical & Pharmaceutical, Taipei, Taiwan).

*Preparation of human peripheral blood mononuclear cells*

Buffy coats from healthy blood donors, screened negative for HIV-1/2, HTLV-I/II, HCV, HBsAg and containing normal levels of alanine transferase (ALT), were obtained from the Tainan Blood Center, Chinese Blood Services Foundation (Tainan, Taiwan). Peripheral blood mononuclear cells (PBMC) were isolated by density centrifugation (400 x g) on Ficoll-Paque (Amersham Biosciences AB, Uppsala, Sweden). The cells were then washed twice in PBS and collected by 100 x g centrifugation.

*Magnetic cell purification*

PBMC were first magnetically labeled with CD45RO MACS microbeads (Miltenyi Biotec, Auburn CA) then separated by using a VarioMACS (Miltenyi Biotec) instrument. Briefly, the cells were specifically labeled with super-paramagnetic MACS microbeads. After magnetic labeling, the cells were passed through a separation column which was placed in a strong permanent magnet. The magnetizable column matrix served to create a high-gradient magnetic field. The magnetically labeled cells were retained in the column and separated from the unlabeled cells, which passed through. After removal of the column from the magnetic field, the retained cells were eluted. The eluted CD45RO$^+$ cells were recovered by 100 x g centrifugation and were used immediately.

*CD45RO$^+$ T cell replacing factors*

The CD45RO$^+$ T cells were cultured in tissue culture flasks at a density of 2 x 10$^6$ cells/ml in RPMI-1640 supplemented with 1 x non-essential amino acids, 10% human serum, 50 mg/ml gentamycin /kanamycin, 50 mM 2-mercaptoethanol and 10 mg/ml pokeweed mitogen (PWM; Sigma Chemicals). After 24 hr incubation, cells were spun down and removed by 400 x g centrifugation. Finally, CD45RO$^+$ T cell replacing factor, *i.e.*, culture supernatant, was prepared by harvesting the culture supernatant, filtering with a 0.45 mm filter, and stored frozen at -20°C.

*Cytotoxic Cell Depletion from PBMC*

Removal of cytotoxic cell populations, which inhibit *in vitro* immunization, was performed by using the L-leucyl-L-leucine methyl ester (LeuLeuOMe) treatment or magnetic cell depletion. The LeuLeuOMe treatment was performed by adding 250 mM L-leucyl-L-leucine methyl ester hydro-bromide (Sigma, St. Louis, MO) to a suspension of PBMC (10$^7$ cells/ml) as previously described (Ohlin et al., 1989; Chin et al., 1994; Chin et al., 1995). Magnetic depletion was performed by using colloidal super-paramagnetic microbeads conjugated to monoclonal anti-mouse CD8 and anti-CD56 antibodies (Miltenyi Biotech) as described above in the *Magnetic cell purification* section.

*In vitro immunization*

PBMC, particularly cytotoxic cell-depleted PBMC, were immunized *in vitro* using a two-step immunization protocol. Primary immunization was performed by incubating the cells for 6 days in a medium containing 10 nM of the peptide antigen (TT-V3B(MN)), 50 mM 2-mercaptoethanol, 10% heat-inactivated human serum, 0.05 ng/ml rIL2 (Calbiochem, San Diego, CA), and 25% (v/v) T cell replacing factor. On day 7, cells from the primary immunization were harvested and spun through 40% Ficoll-Paque. For secondary immunization, 3 x 10$^7$ cells were mixed with the peptide antigen in a flask that had been immobilized overnight with 5mg/ml of CD40L (CD154; Vinci-Biochem, Vinci, Italy). The cells were cultured for 3-5 days in a medium supplemented with 5% human serum, 50 mM 2-mercaptoethanol and 10 nM peptide antigen.

*Epstein-Barr virus (EBV) infection*

The *in vitro* immunized cells were infected with EBV. Briefly, 10$^7$ lymphocytes were incubated for 2 hr at 37°C with occasional resuspension with 1 ml EBV-containing supernatant derived from the EBV-producing marmoset cell line B95-8 (American Type Culture Collection, ATCC CRL 1612; kindly provided by Dr. L.-F. Shu, Tri Services General Hospital, Taipei). The infected cells were seeded at 10$^5$/well in 96-well plates together with mytomycin (Kyowa Hakko Kogyo, Toyoko, Japan)-treated PBMC as feeder cells (10$^4$/well).

*Somatic cell hybridization by electrofusion*

Somatic cell hybridization was performed by electrofusion as previously described (Chin et al., 1994; Chin et al., 1995). Briefly, lymphoblastoid cells were fused with heteromyeloma (Chin et al., 2001) cells at a ratio of 2 heteromyeloma cells to 1 human lymphoblastoid cell in 24-well tissue culture plates (Nalge Nunc International, Roskilde, DK) in an isotonic medium (280 mM sorbitol, 0.5 mM magnesium acetate, 0.1 mM calcium acetate and 1mg/ml BSA; pH6.9-7.1). Cell fusion was performed for 30 seconds at 38 V (200 V/cm) followed by three pulses of 15-microsecond duration at 285 V (1500 V/cm) using a BTX Electro Cell Manipulator Mode 200. The cells were left in the microcuvette for 20-30 min after fusion, then gently resuspended in a culture medium supplemented with 10% FCS. Antigen-specific hybrids were selected and cloned as described in Chin et al., 2001.

*Conjugation of peptide antigens and carrier*

Synthetic peptides as shown in Tables 1 and 2 were used as assay antigens for ELISA. Each of the peptides was lyophilized and conjugated with BSA by a two-step reaction using Sulfo-MBS (Pierce, Rockford, IL) as a crosslinking agent. First, BSA was reconstituted with 2 ml distilled water to yield a 10 mg/ml solution. 200 µl of reconstituted BSA was mixed with 100 µl of Sulfo-MBS solution (2 mg/ml) in a test tube and stirred for 1 hr at room temperature. The maleimide-activated BSA (~100 µl) was purified on Sephadex G-10 columns to remove excess crosslinker. Thereafter, 500 µl of a sulfhydryl-containing peptide in conjugation buffer (4 mg/ml) was added, and the mixture was incubated for 2 hrs at room temperature. The reaction product was dialyzed, and then lyophilized for further use. For ELISA, the conjugate (2 mg/ml) was added to 96-well microtiter plates at 100 µl/well and the plates were incubated at 4°C overnight for immobilization of the antigen.

*Enzyme-linked immunosorbent assay (ELISA)*

Antigen-specific ELISA was performed by first coating 1 mg/ml baculovirus-expressed recombinant HIV-1 IIIB or MN gp120 (ImmunoDiagnostics, Woburn, MA), 5 mg/ml human transferrin (Sigma), 10 mg/well of bovine serum albumin (BSA; Sigma) or tetanus toxoid (ADImmune Corporation, Taichung, Taiwan) onto microtitre plates overnight at room temperature. Culture supernatants were diluted to the desired level in 10 mM sodium phosphate buffer, pH 8.0, containing 0 5 M sodium chloride and 0.1% Tween-20. Coated plates were incubated with diluted culture supernatants, washed, incubated with peroxidase-labeled goat antibodies against human IgG and IgM (Zymed Laboratories, So. San Francisco, CA) and developed (15 min) by addition of 100 µl of the chromogenic substrate o-phenylaenediamine (OPD) (Sigma). The reaction was stopped after 30 min by adding 1 M sulphuric acid, and the absorbances were read at 490 nm.

*Competitive ELISA*

The specificity of huMAb was confirmed by a competitive inhibition ELISA. Briefly, 01-100 mM peptide antigens were incubated with 1 nM of the huMAb of interest at 4°C overnight in a dilution buffer (0.5 M sodium chloride and 0.1% Tween 20, pH 8.0). The mixtures were transferred to ELISA plates coated with recombinant gp120. After 2 hr of incubation in 37 °C, the amount of gp120-specific huMAb bound to the plates was evaluated using peroxidase-labeled goat antibodies specific for human IgG (Zymed Laboratories, So. San Francisco, CA).

*HIV-1 neutralization*

The huMab to be tested was combined at 20 µl/well in 96-well U-bottom plates (Nalge Nunc International) with 40 µl of IL-2 culture medium/well. The huMAb may be first diluted if desired. 14 µl of HIV-1 MN or IIIB virus (500 to 2500 TCID50) was then added to each well and incubated at 37°C for 1 hr. Afterwards, 100 µl of PBMC (containing 500,000 viable cells) was added to each well and incubated at 37°C overnight. The culture was then washed three times with the same media to remove the virus from the media. On days 4 (from addition of PBMC), 25 µl of culture supernatant was transferred to the corresponding well of a fresh 96-well plate for the measurement of reverse transcriptase (RT) activity by a non-radioactive method (RetroSys™ Innovagen AB, Lund, Sweden). The reduction in RT activity was calculated relative to a negative control, a human myeloma IgG1k (The Binding Site Limited, Birmingham, UK) that was diluted in the same manner as the huMab.

**EXAMPLE 1**

**PREPARATION OF PEPTIDE ANTIGENS**

Synthetic peptides corresponding to the V3 loop of HIV-1 gp120 are shown in Table 1.

## TABLE 1

| Synthetic peptides | HIV-1 strains / subtypes | Amino acid sequence and alignment | SEQ ID No. |
|---|---|---|---|
| | | Amino acid sequences of synthetic peptides within the V3 coreceptor-binding region of HIV-1 gp120 | |
| $V3_{ALL}con$ | HIV-1 consensus | N' — R K S I H I . . G P G Q A F Y A T — C' | 2 |
| $V3_{A}con$ | Subtype A consensus | N' — - - - V R - . . - - - - - - - - - — C' | 3 |
| $V3_{B}con$ | Subtype B consensus | N' — - - - - - - . . - - - R - - - T - — C' | 4 |
| $V3_{B(MN)}$ | MN (B subtype) | N' — - - R - - - . . - - - R - - - T - — C' | 5 |
| $V3_{B(IIIB)}$ | IIIB (B subtype) | N' — - - - - R - Q R - - - R - - V — C' | 6 |
| $V3_{C}con$ | Subtype C consensus | N' — - - - - R - . . - - - - T - - - - — C' | 7 |
| $V3_{D}con$ | Subtype D consensus | N' — - Q - T - - . . - - - - L - - - — C' | 8 |
| $V3_{E}con$ | Subtype E consensus* | N' — - T - - T - . . - - - V - - R - — C' | 9 |
| $V3_{F}con$ | Subtype F consensus | N' — - - - - - L . . - - - - - - - - — C' | 10 |
| $V3_{G}con$ | Subtype G consensus | N' — - - - - R - . . - - - - - - - - — C' | 11 |
| $V3_{H}con$ | Subtype H consensus | N' — - - - - - - . . - - - - - - - - — C' | 2 |
| $V3_{J}con$ | Subtype J consensus | N' — - - G - - M . . - - - V L - - - — C' | 12 |
| $V3_{K}con$ | Subtype K consensus | N' — - - - - - - . . - - R - - - - - — C' | 13 |
| $V3_{O1}con$ | Subtype O1 consensus | N' — - T - - T - . . - - - V - - R - — C' | 14 |
| $V3_{O2}con$ | Subtype O2 consensus | N' — - - - V R - . . - - - T - - - - — C' | 15 |
| $V3_{O3}con$ | Subtype O3 consensus | N' — - - G - - - . . - - R - - - - - — C' | 16 |
| $V3_{O4}con$ | Subtype O4 consensus | N' — - - - V - - . . - - - T W - - - — C' | 17 |

Amino acid residues and sequences were obtained from the Los Alamos National Laboratory (Los Alamos, NM; see Korber et al., 2001), except the one of Subtype E consensus* was adopted from Shiino et al., 2000. A dash (-) indicates concurrence with the top sequence in the alignment and a period (.) indicates a deletion.

EP 1 498 426 A1

Peptide sequences derived from the V3 loop of HIV-1 gp120 are shown in Table 2. For example, to generate an immunogen containing both T-cell and B-cell epitopes, the "helper" sequence derived from tetanus toxin encompassing amino acids 830-844 (see peptide "TT", SEQ ID NO: 20 in Table 2; Chin et al., 1994; Chin et al., 1995; Demotz et al., 1989) was combined with a fragment of V3 of HIV-1 MN strain (peptide "V3$_{B(MN)}$", SEQ ID NO:6 in Table 1) to form TT-V3$_{B(MN)}$ (Table 2, SEQ ID NO:1).

## TABLE 2

| | Amino acid sequences of synthetic peptides derived from the V3 loop of HIV-1 gp120 | |
|---|---|---|
| Synthetic peptides | Amino acid sequence | SEQ ID No. |
| TT-V3$_{B(MN)}$ | N'—QYIKANSKFIGITELRKRIHIGPGRAFYTT—C' | 1 |
| V3-C4$_D$con | N'—RQSTHIGPGQALYTTKDIIGDIRQAHCNISGAEWN—C' | 18 |
| V3-C4$_{OI}$con | N'—RTSITIGPGQVFYRTGDIIGDIRKAYCEINGTKWN—C' | 19 |
| TT | N'—QYIKANSKFIGITEL—C' | 20 |

## EXAMPLE 2

### REMOVAL OF THE CD8$^+$ AND CD56$^+$ POPULATIONS RESULTED IN INCREASED ANTIGEN RESPONDERS

Since removal of cytotoxic or inhibitory cell populations plays an integral role in antigen responsiveness *in vitro* (Ohlin et al., 1989; 1992), the effects of different reagents that may be useful in removing cytotoxic cells were compared in *in vitro* immunization studies. These reagents include LeuLeuOMe, magnetic cell depletion using anti-CD8 antibodies, magnetic cell depletion using anti-CD56 antibodies, and magnetic cell depletion using a combination of anti-CD8 antibodies and anti-CD56 antibodies. Thus, peripheral blood mononuclear cells (PBMCs) were subject to each of the reagents or, as a control, no additional reagent (nil). The PBMCs from four different donors were tested. *In vitro* immunization was performed as described in Materials and Methods, including primary and secondary immunization against the peptide antigen TT-V3B(MN). To prolong lives of the immunized cells, the cells were then infected with EBV as described above. The efficacy of each treatment was evaluated based on the specific efficiency, *i.e.*, the number of wells in the culture plates that secreted antibodies specific for the antigen. TT-V3B(MN).
As shown in Table 3, removal of CD8 or CD56 cells resulted in increased specific efficiency. When both CD8 and CD56 cells were removed, the specific efficiency was even higher. The effect was superior to that of LeuLeuOMe treatment, which is the most commonly used method. With no treatment (nil), no antibody production was scored. Therefore, these results demonstrate that removal of CD8 and CD56 cells significantly improved the efficiency *of in vitro* immunization.

Table 3

| Donor | Specific efficiency of *in vitro* stimulation using peptide antigen TT-V3$_{B(MN)}$ | | | | | | | | | |
|---|---|---|---|---|---|---|---|---|---|---|
| | Number of wells with specific antibody production* | | | | | | | | | |
| | Nil | | LeuLeuOMe | | CD8 removal | | CD56 removal | | CD8& CD56 removal | |
| A | 0/47 | (0%) | 0/75 | (0%) | 1/70 | (1.43%) | 0/70 | (0%) | 5/75 | (6.66%) |
| B | 0/47 | (0%) | 0/72 | (0%) | 0/77 | (0%) | 1/96 | (1.04%) | 2/96 | (2.08%) |
| C | 0/47 | (0%) | 3/80 | (3.75%) | 3/76 | (3.95%) | 0/53 | (0%) | 3/77 | (3.90%) |
| D | 0/47 | (0%) | 0/68 | (0%) | 0/81 | (0%) | 0/46 | (0%) | 2/52 | (3.85%) |

Specific efficiency was defined as:

$$\frac{\text{Number of wells containing specific antibody production}}{\text{Number of wells containing growing lymphoblastoid cells after EBV activation and } \textit{in vitro} \text{ immunization}}$$

*A well containing lymphoblastoid cells was scored as specific antibody-producing if:

   1. the ELISA OD value against recombinant gp120(IIIB) was at least five times as high as the OD value for the negative control;

   2. the reactivity index (RI) was >2, where RI = [OD$_{gp120(IIIB)}$ - OD$_{medium\ control\ against\ gp120(IIIB)}$] / [OD$_{BSA}$ - OD$_{medium\ control\ against\ BSA}$].

EP 1 498 426 A1

**EXAMPLE 3**

**Preparation of antibodies that recognize multiple antigens**

One of the major unresolved problems in the field of HIV treatment is how to develop a single reagent that neutralizes a broad range of HIV variants. Most antibodies reported to date that recognize the co-receptor binding region are strain-specific. The present invention, on the other hand, provides a novel method for immunization which leads to the production of anti-HIV antibodies that recognize conformational epitopes in the co-receptor binding region. The antibodies thus recognize multiple stains of HIV. This method can be used to prepare broad-spectrum antibodies that recognize other antigens/microorganisms as well.

In this example, PBMC were immunized with the co-receptor binding region of one strain of HIV-1 and subsequently screened by ELISA with an antigen derived from another HIV strain which has a different amino acid sequence in the co-receptor binding region. Thus, a peptide synthesized based on the amino acid sequence of the gp120 region of HIV-1 MN strain, TT-v$_{3B(MN)}$ (see Table 2), was used for immunization, while a recombinant gp120 sequence derived from the IIIB strain with a different primary structure (see Table 1) was used for screening.

Antibody-producing cells, which produce antibodies that recognize gp 120 of both strains, were obtained with such an immunization/screening procedure. The antibodies were characterized as described in Example 4.

**EXAMPLE 4**

**Production and characterization of a specific trioma cell line stably secreting human IgG1**

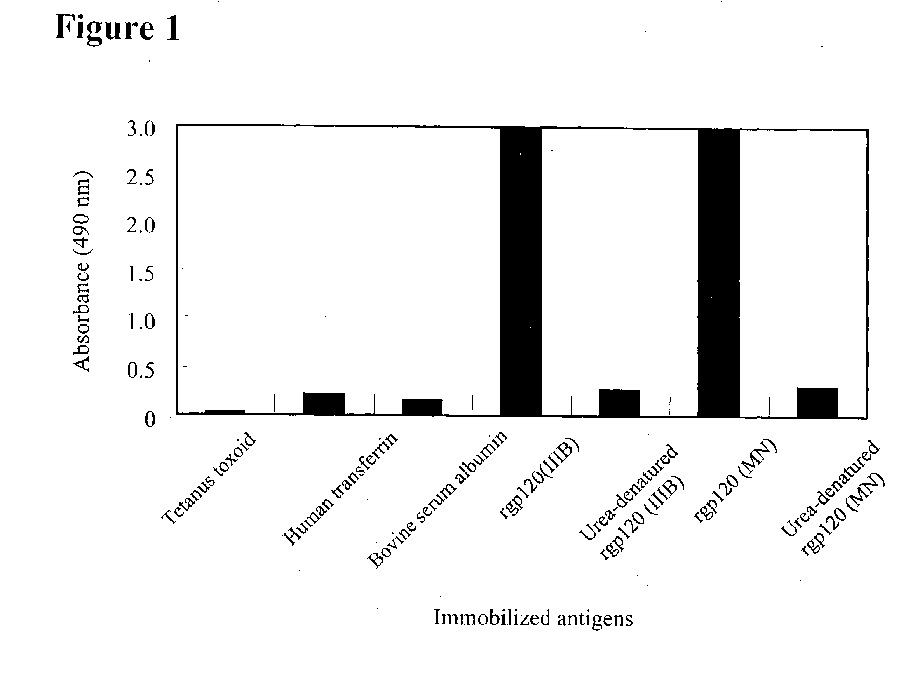

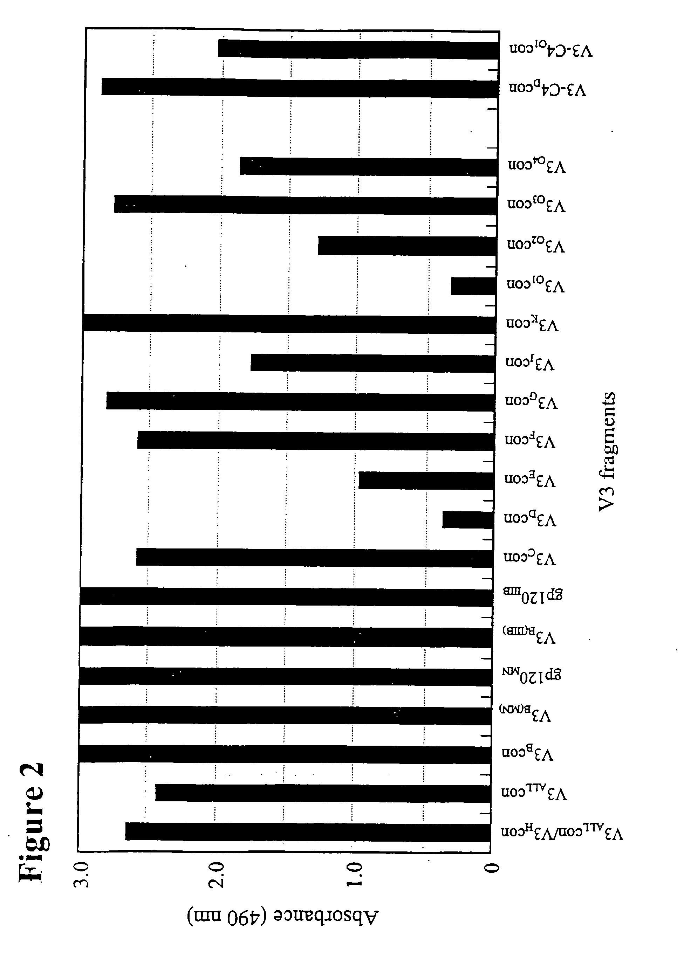

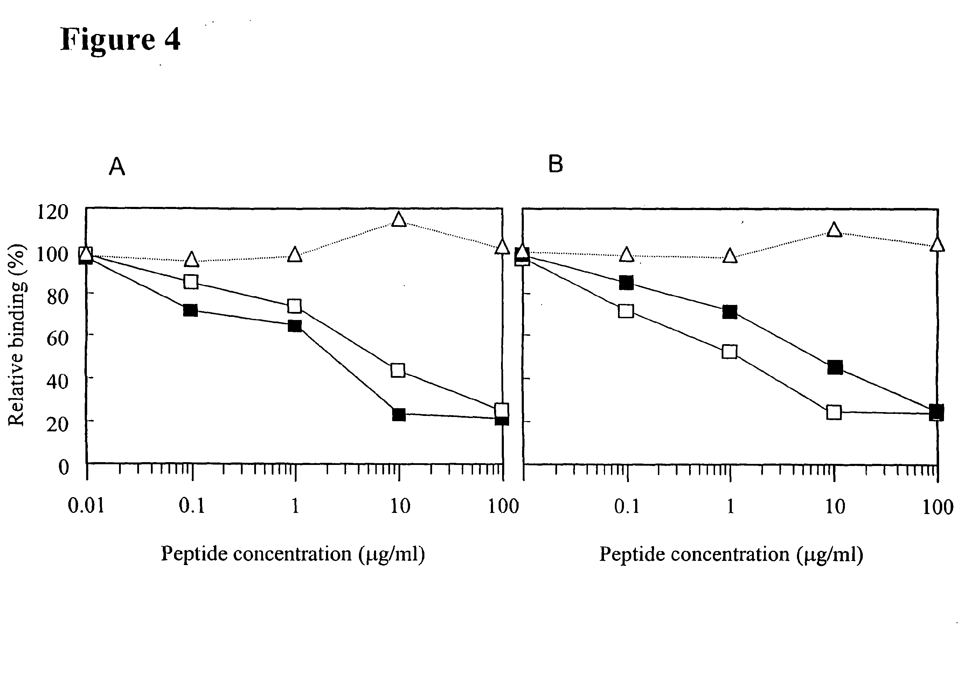

To immortalize the antibody-producing cells generated *in vitro* in the Example above for long-term antibody production, the cells were fused with a heteromyeloma cell line. A stable (for more than two years) human trioma cell line was established after cloning and screening. The monoclonal antibody produced from this cell line is designated LTC-gp120-IgG1k. This huMAb was of IGg1 isotype with k light chain and the productivity was 1 mg/($10^6$ cells x 24 hr). Figure 1 demonstrates the specificity of LTC-gp120-IgG1k. The antibody reacted strongly with both gp120(IIIB) and gp120(MN), but it showed only background level reactivities with unrelated antigens, such as tetanus toxoid, transferrin and BSA. Moreover, the antibody displayed a much reduced reactivity with denatured gp120, of either IIIB or MN origin. Since only linear epitopes may be recognized in a denatured protein, these results indicate that LTC-gp120-IgG1k reacts with a conformational epitope commonly found in gp120 of several HIV-1 strains. In fact, with the exception of D and O1 subtypes, this huMAb recognized the V3 consensus peptides of all the HIV-1 strains listed in Table 1. Moreover, the reduced response observed for the D and O1 subtype peptides could be rescued by adding parts of the C4 region to the peptides. confirming that the huMAb binds to the co-receptor binding site. The specificity of the antibody was also verified with competitive ELISA, as shown in Figure 4.

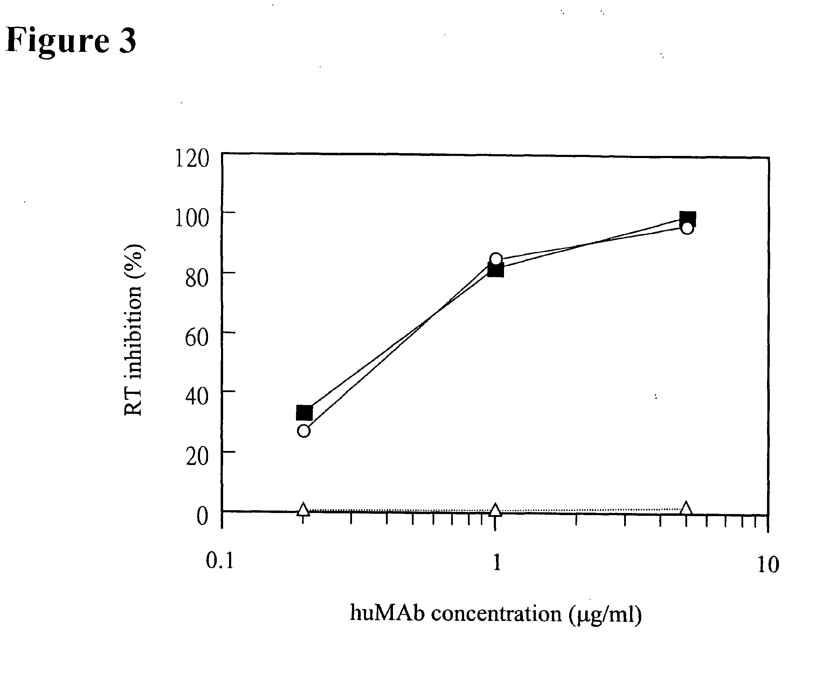

LTC-gp120-IgG1k neutralized HIV-1 IIIB and MN in a dose-dependent manner. The virus was cultured under conditions that it could infect host cells, and the antibody was added to the culture to determine its effect on viral activity. The viral reverse transcriptase activity was measured as an indication of infectivity of the virus. As shown in Figure 3, a concentration of 0.38 µg/ml of the antibody resulted in 90% neutralization of the reverse transcriptase activity of the virus. Therefore, the antibody will be an effective therapeutic or prophylactic agent against HIV infection.

**Claims**

1. A method of preparing a fully human antibody recognizing an antigen, comprising:

(a) providing a group of lymphocytes from a naïve human donor;
(b) immunizing said lymphocytes with the antigen *in vitro;*
(c) fusing the immunized lymphocytes with a heteromyeloma cell line to form trioma cells;
(d) identifying trioma cells that produce an antibody that recognizes the antigen; and
(e) collecting the antibody produced by the trioma cells identified in step (d).

2. The method of claim 1, further comprising the step of removing CD8$^+$ cells and CD56$^+$ cells from said lymphocytes prior to step (b).

3. The method of claim 1 or 2, further comprising screening the trioma cells of step (c) with a second antigen prior to step (d), thereby selecting cells that produce antibodies which recognize both the antigen and the second antigen.

4. The method of any of the above claims wherein the antibody recognizes the antigen with a Kd of about 30 nM or less.

5. The method of any of the above claims wherein the antibody is an IgG antibody.

6. The method of any of the above claims wherein the antibody is an IgG1 antibody.

7. The method of any of the above claims wherein the trioma cells of step (d) are capable of producing the antibody for at least about 3 months in cell culture.

8. The method of any of the above claims wherein the trioma cells of step (d) are capable of producing the antibody for at least about 6 months in cell culture.

9. The method of any of the above claims wherein the trioma cells of step (d) are capable of producing the antibody for at least about 9 months in cell culture.

10. The method of any of the above claims wherein the trioma cells of step (d) are capable of producing the antibody for at least about 12 months in cell culture.

11. The method of any of the above claims wherein the antigen is an HIV antigen.

12. The method of claim 11 wherein the antigen is derived from gp120.

13. The method of claim 12 wherein the antigen comprises the co-receptor binding region of gp120.

14. The method of any of the above claims wherein the antigen comprises a T-helper sequence.

15. An isolated fully human antibody, or an antigen-binding fragment thereof, wherein the antibody recognizes at least two strains of HIV.

16. The antibody or fragment of claim 15 that recognizes the gp120 of at least two strains of HIV.

17. The antibody or fragment of claim 16 that recognizes the co-receptor binding region of gp120.

18. The antibody or fragment of claim 15 that recognizes at least two sequences selected from the group consisting of SEQ ID Nos:2-17.

19. The antibody or fragment of any of claims 15-18 wherein the antibody is an IgG.

20. The antibody or fragment of any of claims 15-18 wherein the antibody is an IgG1.

21. A composition comprising the antibody or fragment of any of claims 15-20.

22. The composition of claim 21 further comprising a pharmaceutically acceptable carrier or excipient.

23. Use of an effective amount of the composition of claim 21 for the preparation of a pharmaceutical composition for preventing, treating or ameliorating an HIV infection of a subject in need thereof.

**24.** The use of claim 23 wherein the subject suffers from AIDS.

**25.** A method of preparing a fully human antibody recognizing at least two different antigens, comprising:

(a) providing a group of lymphocytes from a naive human donor;
(b) immunizing said lymphocytes with a first antigen *in vitro*;
(c) fusing the immunized lymphocytes with a heteromyeloma cell line to form trioma cells;
(d) screening the trioma cells with a second antigen to identify cells that produce antibodies which recognize both the first antigen and the second antigen; and
(e) collecting the antibody produced by the trioma cells identified in step (d).

**26.** The method of claim 25 wherein the first antigen and the second antigen are from a microorganism.

**27.** The method of claim 25 wherein the first antigen and the second antigen are from two different strains of a microorganism.

**28.** The method of claim 27 wherein the microorganism is HIV.

**29.** The method of claim 28 wherein the first antigen and the second antigen are derived from gp 120.

**30.** A method of increasing the efficiency of *in vitro* immunization of lymphocytes with an antigen, comprising:

(a) providing a population of lymphocytes;
(b) removing $CD8^+$ and $CD56^+$ cells from said population; and
(c) contacting said population of lymphocytes with the antigen *in vitro.*

**31.** The method of claim 30 wherein the $CD8^+$ and $CD56^+$ cells are removed by using magnetic beads specific for CD8 and CD56.

**32.** An *in vitro* cell population prepared by a method comprising:

(a) providing peripheral blood mononuclear cells from a naive human donor;
(b) removing $CD8^+$ and $CD56^+$ cells from said peripheral blood mononuclear cells; and
(c) contacting the cells of step (b) with an antigen *in vitro,* resulting in production by the cells of antibodies that recognize said antigen.

**33.** An antibody-producing cell prepared by culturing the cell population of claim 32 under clonal conditions and isolating clones that produce antibodies that recognize said antigen.

**34.** The antibody-producing cell of claim 33 that produces antibodies that recognize HIV gp 120.

**35.** The antibody-producing cell of claim 34 which produces antibodies that recognize at least two gp120 molecules derived from different strains of HIV.

## Figure 1

# Figure 2

# Figure 3

# Figure 4

EP 1 498 426 A1

**European Patent Office**

**EUROPEAN SEARCH REPORT**

Application Number

EP 04 01 6838

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | CHIN L T ET AL: "Mimicking the humoral immune response in vitro results in antigen-specific isotype switching supported by specific autologous T helper cells: generation of human HIV-1-neutralizing IgG monoclonal antibodies from naive donors." EUROPEAN JOURNAL OF IMMUNOLOGY. MAR 1995, vol. 25, no. 3, March 1995 (1995-03), pages 657-663, XP009038578 ISSN: 0014-2980 * the whole document * | 1-22,25, 26,30, 32-35 | C07K16/00 C12N5/00 |
| X | CHIN L -T ET AL: "Site-directed primary in vitro immunization: Production of HIV-1 neutralizing human monoclonal antibodies from lymphocytes obtained from seronegative donors" IMMUNOLOGY, vol. 81, no. 3, 1994, pages 428-434, XP009038559 ISSN: 0019-2805 * the whole document * | 1,4, 7-18,21, 22 | |
| X | ZWICK MICHAEL B ET AL: "A novel human antibody against human immunodeficiency virus type 1 gp120 is V1, V2, and V3 loop dependent and helps delimit the epitope of the broadly neutralizing antibody immunoglobulin G1 b12." JOURNAL OF VIROLOGY, vol. 77, no. 12, June 2003 (2003-06), pages 6965-6978, XP002302361 ISSN: 0022-538X * abstract * | 15-18, 21,22 | TECHNICAL FIELDS SEARCHED (Int.Cl.7) C07K C12N |

-/--

The present search report has been drawn up for all claims

| Place of search | Date of completion of the search | Examiner |
|---|---|---|
| Munich | 27 October 2004 | Lechner, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 498 426 A1

<table>
<tr><td rowspan="2">European Patent Office</td><td rowspan="2">EUROPEAN SEARCH REPORT</td><td>Application Number</td></tr>
<tr><td>EP 04 01 6838</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DERCAMP CHRISTOPHE ET AL: "Depletion of human NK and CD8 cells prior to in vitro H1N1 flu vaccine stimulation increases the number of gamma interferon-secreting cells compared to the initial undepleted population in an ELISPOT assay" CLINICAL AND DIAGNOSTIC LABORATORY IMMUNOLOGY, vol. 9, no. 2, March 2002 (2002-03), pages 230-235, XP002302365 ISSN: 1071-412X * abstract * | 30-32 | |
| X | WO 01/00678 A (REITZ MARVIN S JR ; WATKINS BRYNMOR A (US); US HEALTH (US)) 4 January 2001 (2001-01-04) * abstract * | 23,24 | |
| A | KOBAYASHI YOSHIRO ET AL: "Cell-type specificity of L-leucyl L-leucine methyl ester" BIOCHEMICAL AND BIOPHYSICAL RESEARCH COMMUNICATIONS, vol. 272, no. 3, 16 June 2000 (2000-06-16), pages 687-690, XP002302363 ISSN: 0006-291X * abstract * * figure 3 * | 1-35 | TECHNICAL FIELDS SEARCHED (Int.Cl.7) |

-/--

The present search report has been drawn up for all claims

| Place of search | Date of completion of the search | Examiner |
|---|---|---|
| Munich | 27 October 2004 | Lechner, O |

**European Patent Office**

## EUROPEAN SEARCH REPORT

Application Number

EP 04 01 6838

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | TRUJILLO J R ET AL: "Shared Antigenic Epitopes on the V3 Loop of HIV-1 gp120 and Proteins on Activated Human T Cells" VIROLOGY, ACADEMIC PRESS,ORLANDO, US, vol. 246, no. 1, 20 June 1998 (1998-06-20), pages 53-62, XP004445774 ISSN: 0042-6822 * abstract * | 1-35 | |
| A | PUHLMANN CLAUS M ET AL: "Optimizing production of human monoclonal IgG antibodies by in vitro-primed human PBMC: Influence of CD56+ NK cell depletion" HYBRIDOMA, vol. 14, no. 4, 1995, pages 391-396, XP009038734 ISSN: 0272-457X * the whole document * | 1-35 | |
| D,A | DUENAS M ET AL: "In vitro immunization of naive human B cells yield high affinity immunoglobulin G antibodies as illustrated by phage display" IMMUNOLOGY, vol. 89, no. 1, 1996, pages 1-7, XP002302364 ISSN: 0019-2805 * abstract * | 1-35 | TECHNICAL FIELDS SEARCHED (Int.Cl.7) |
| A | OHLIN M ET AL: "HUMAN MOABS PRODUCED FROM NORMAL HIV-1-NEGATIVE DONORS AND SPECIFIC FOR GLYCOPROTEIN GP120 OF THE HIV-1 ENVELOPE" CLINICAL AND EXPERIMENTAL IMMUNOLOGY, vol. 89, no. 2, 1992, pages 290-295, XP009038562 ISSN: 0009-9104 * abstract * | 1-35 | |

The present search report has been drawn up for all claims

| Place of search | Date of completion of the search | Examiner |
|---|---|---|
| Munich | 27 October 2004 | Lechner, 0 |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 04 01 6838

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2004

| Patent document cited in search report | | Publication date | Patent family member(s) | | Publication date |
|---|---|---|---|---|---|
| WO 0100678 | A | 04-01-2001 | AU | 5761900 A | 31-01-2001 |
| | | | WO | 0100678 A1 | 04-01-2001 |

For more details about this annex : see Official Journal of the European Patent Office, No. 12/82

SEQUENCE LISTING

<110> Chin, Li-Te

<120> PREPARATION OF FULLY HUMAN ANTIBODIES

<130> 16863-002001

<140> US 10/622,003
<141> 2003-07-16

<160> 20

<170> FastSEQ for Windows Version 4.0

<210> 1
<211> 30
<212> PRT
<213> Artificial Sequence

<220>
<223> Synthetically generated peptide

<400> 1
Gln Tyr Ile Lys Ala Asn Ser Lys Phe Ile Gly Ile Thr Glu Leu Arg
1               5                   10                  15
Lys Arg Ile His Ile Gly Pro Gly Arg Ala Phe Tyr Thr Thr
            20                  25              30

<210> 2
<211> 15
<212> PRT
<213> Artificial Sequence

<220>
<223> Consensus sequence

<400> 2
Arg Lys Ser Ile His Ile Gly Pro Gly Gln Ala Phe Tyr Ala Thr
1               5                   10                  15

<210> 3
<211> 15
<212> PRT
<213> Artificial Sequence

<220>
<223> Consensus sequence

<400> 3
Arg Lys Ser Val Arg Ile Gly Pro Gly Gln Ala Phe Tyr Ala Thr
1               5                   10                  15

<210> 4
<211> 15
<212> PRT
<213> Artificial Sequence

<220>
<223> Consensus sequence

<400> 4
Arg Lys Ser Ile His Ile Gly Pro Gly Arg Ala Phe Tyr Thr Thr
1               5                   10                  15

<210> 5
<211> 15
<212> PRT
<213> Artificial Sequence

```
<220>
<223> Synthetically generated peptide

<400> 5
Arg Lys Arg Ile His Ile Gly Pro Gly Arg Ala Phe Tyr Thr Thr
 1               5               10              15

<210> 6
<211> 15
<212> PRT
<213> Artificial Sequence

<220>
<223> Synthetically generated peptide

<400> 6
Arg Lys Ser Ile Arg Ile Gln Arg Gly Pro Gly Arg Ala Phe Val
 1               5               10              15

<210> 7
<211> 15
<212> PRT
<213> Artificial Sequence

<220>
<223> Consensus sequence

<400> 7
Arg Lys Ser Ile Arg Ile Gly Pro Gly Gln Thr Phe Tyr Ala Thr
 1               5               10              15

<210> 8
<211> 15
<212> PRT
<213> Artificial Sequence

<220>
<223> Consensus sequence

<400> 8
Arg Gln Ser Thr His Ile Gly Pro Gly Gln Ala Leu Tyr Ala Thr
 1               5               10              15

<210> 9
<211> 15
<212> PRT
<213> Artificial Sequence

<220>
<223> Consensus sequence

<400> 9
Arg Thr Ser Ile Thr Ile Gly Pro Gly Gln Val Phe Tyr Arg Thr
 1               5               10              15

<210> 10
<211> 15
<212> PRT
<213> Artificial Sequence

<220>
<223> Consensus sequence

<400> 10
Arg Lys Ser Ile His Leu Gly Pro Gly Gln Ala Phe Tyr Ala Thr
 1               5               10              15

<210> 11
<211> 15
<212> PRT
```

18

```
<213> Artificial Sequence

<220>
<223> Consensus sequence

<400> 11
Arg Lys Ser Ile Arg Ile Gly Pro Gly Gln Ala Phe Tyr Ala Thr
 1               5                   10                  15

<210> 12
<211> 15
<212> PRT
<213> Artificial Sequence

<220>
<223> Consensus sequence

<400> 12
Arg Lys Gly Ile His Met Gly Pro Gly Gln Val Leu Tyr Ala Thr
 1               5                   10                  15

<210> 13
<211> 15
<212> PRT
<213> Artificial Sequence

<220>
<223> Consensus sequence

<400> 13
Arg Lys Ser Ile His Ile Gly Pro Gly Arg Ala Phe Tyr Ala Thr
 1               5                   10                  15

<210> 14
<211> 15
<212> PRT
<213> Artificial Sequence

<220>
<223> Consensus sequence

<400> 14
Arg Thr Ser Ile Thr Ile Gly Pro Gly Gln Val Phe Tyr Arg Thr
 1               5                   10                  15

<210> 15
<211> 15
<212> PRT
<213> Artificial Sequence

<220>
<223> Consensus sequence

<400> 15
Arg Lys Ser Val Arg Ile Gly Pro Gly Gln Thr Phe Tyr Ala Thr
 1               5                   10                  15

<210> 16
<211> 15
<212> PRT
<213> Artificial Sequence

<220>
<223> Consensus sequence

<400> 16
Arg Lys Gly Ile His Ile Gly Pro Gly Arg Ala Phe Tyr Ala Thr
 1               5                   10                  15

<210> 17
<211> 15
```

```
<212> PRT
<213> Artificial Sequence

<220>
<223> Consensus sequence

<400> 17
Arg Lys Ser Val His Ile Gly Pro Gly Gln Thr Trp Tyr Ala Thr
 1               5                   10                  15

<210> 18
<211> 35
<212> PRT
<213> Artificial Sequence

<220>
<223> Synthetically generated peptide

<400> 18
Arg Gln Ser Thr His Ile Gly Pro Gly Gln Ala Leu Tyr Thr Thr Lys
 1               5                   10                  15
Asp Ile Ile Gly Asp Ile Arg Gln Ala His Cys Asn Ile Ser Gly Ala
            20                  25                  30
Glu Trp Asn
        35

<210> 19
<211> 35
<212> PRT
<213> Artificial Sequence

<220>
<223> Synthetically generated peptide

<400> 19
Arg Thr Ser Ile Thr Ile Gly Pro Gly Gln Val Phe Tyr Arg Thr Gly
 1               5                   10                  15
Asp Ile Ile Gly Asp Ile Arg Lys Ala Tyr Cys Glu Ile Asn Gly Thr
            20                  25                  30
Lys Trp Asn
        35

<210> 20
<211> 15
<212> PRT
<213> Artificial Sequence

<220>
<223> Synthetically generated peptide

<400> 20
Gln Tyr Ile Lys Ala Asn Ser Lys Phe Ile Gly Ile Thr Glu Leu
 1               5                   10                  15
```